# EUROPEAN PATENT SPECIFICATION

(11) **EP 3 808 850 B1**
(45) Date of publication and mention of the grant of the patent: **12.01.2022**
(21) Application number: 20180783.1
(22) Date of filing: 18.06.2020
(51) Int. Cl.: C12P 5/02, C12N 1/38

(54) **METHOD OF ANAEROBIC DIGESTION OF FOOD WASTE BY A COMPOSITION OF BACTERIA**
VERFAHREN ZUR ANAEROBEN VERGÄRUNG VON SPEISERESTEN DURCH EINE BAKTERIENZUSAMMENSETZUNG
PROCÉDÉ DE DIGESTION ANAÉROBIE DE DÉCHETS ALIMENTAIRES PAR UNE COMPOSITION DE BACTÉRIES

(30) Priority: 23.07.2019 LV 190038
(43) Date of publication of application: 21.04.2021
(73) Proprietor: Recolo, SIA, Riga 1063 (LV)
(72) Inventor: Vegere, Kristine, Riga, 1064 (LV); Salava, Elina, Sigulda, 2150 (LV)
(74) Representative: Fortuna, Jevgenijs

(56) References cited:
- WO-A1-2018/136017
- DATABASE EPODOC [Online] 20 October 2010 (2010-10-20), LI, D. ET AL: "Compound microbial bacterial preparation and application thereof", XP055780262, Database accession no. CN101864362 -& CN 101 864 362 A (NANJING CASCADE CLEAN ENERGY RES AND DEV CO LTD) 20 October 2010 (2010-10-20)
- DATABASE WPI Week 201726 Thomson Scientific, London, GB; AN 2017-15906P XP002798940, -& CN 106 434 761 A (UNIV LANZHOU) 22 February 2017 (2017-02-22)
- CHEN, G. ET AL.: "Experimental co-digestion of corn stalk and vermicompost to improve biogas production", WASTE MANAGEMENT, vol. 30, no. 10, 8 April 2010 (2010-04-08) , pages 1834-1840, XP027286115,

## Description

### Technical Field

The invention relates to food waste processing and production of biogas.

### Background Art

Food waste is a significant unused energy source in the modern world. In the majority of households and catering companies food surpluses are the major waste stream. The average yearly production of food waste (FW) is 173 kg per person in Europe (Stenmarck, Å., Jensen, C., Quested, T., Moates, G., 2016. Estimates of European food waste levels. Report of the project FUSIONS granted by the European Commission (FP7)). The food waste has a negative impact on the environment and circular economy. As awareness of this problem is grown, the polices have emerged in recent years. Organic waste landfilling bans are one category of policy that has emerged in Europe to address food waste disposition. However, waste management industries have lack of affordable technologies for the recycling of organic wastes, usually because of low-quality sorted food waste as outcome from centralised sorting facilities. There is increasing awareness for collecting FW as a separate waste stream for production of energy through anaerobic digestion.

It is known that various wastes from food industry such as organic matter rich substrates could be used to produce biogas. However, using traditional methods of processing food industry waste, due to low digestion rates - only till 50-60% of organic matter is degraded, while the other organic part remains in effluent.

Biogas formation process is a complex microbiological process requiring combined activity of several groups of microorganisms with different metabolic capacities and parameters. There is a sequence of microbial events that occur during the digestion process and the production of methane, which are hydrolysis, acid forming, and methanogenesis.

Anaerobic digestion is controlled biological digestion process which allows produce biogas (approx. 60% methane and 40% carbon dioxide) for energy generation. Anaerobic digestion of food waste is achievable but different types, composition of food waste results in varying degrees of methane yields.

The anaerobic digestion process proceeds efficiently if the digestion rates of all three stages are equal (Rugele, K., Skripsts, E., Juhna, T., Larsona, S. Bioaugmentation to Improve Biogas Yield in Cheese Whey Anaerobic Digestion. 13th World Congress on Anaerobic Digestion, Spain, Santiago de Compostella, 25-28 June, 2013).

There are different factors which affect anaerobic digestion efficiency such as environmental conditions like pH, temperature, inhibitory parameters like high organic loading, etc. Volatile solids input, digester temperature and retention time are operational parameter that have a strong effect on biogas production process performance (T. Forster-Carneiro, M. Pérez, and L. I. Romero, "Anaerobic digestion of municipal solid wastes: dry thermophilic performance.," Bioresource technology, vol. 99, no. 17, pp. 8180-4, 2008).

To stabilize quality of the biogas obtained some prior art solutions suggest using microbial consortiums when processing food waste.

There is known a method for increasing methane yield in anaerobic digesters with a cellulolytic bacteria cocktail prepared with rumen fluid obtained from animals (e.g. sheeps, goats) or plants (e.g. sorghum plant) said fluid i.a. comprising *Bacteroidales, Lachnospiraceae, Ruminococcaceae, Firmicutes, Synergistales* bacteria (WO2018136017A1, WO2018136018A1, WO2018136019A1).

There is known a thermophilic microbial consortium for conversion of cellulosic or lignocellulosic biomass to methane, the consortium comprising: a cellulolytic thermophile; an acetate-oxidizing thermophile effective to oxidize acetate to carbon dioxide and hydrogen; and a hydrogen-utilizing thermophilic methanogen (US2011306089).

There is known a method for increasing methane gas generation by adding Fibrobacter succinogenes which is cellulolytic bacteria during anaerobic digestion (KR20130130098A). The method for increasing methane gas generation using cellulolytic bacteria in anaerobic digestion according to the known solution is characterized by increasing methane gas generation by adding Fibrobacter succinogenes which is cellulolytic bacteria to the anaerobic digestion of organic polymer waste.

The main disadvantages of the known methods are relatively insufficient treatment efficiency, high organic and low methane content in output as well as long hydraulic retention time.

### Summary Of The Invention

Disclosed are a composition and a method for increasing biogas production yield in anaerobic digester. The composition comprises a biological agent comprising Cellulolytic bacteria and enzymes. The composition may further comprise one or more biostimulants. The biological agent comprising Cellulolytic bacteria consists of cultures *Pseudomonas koreensis, Pseudomonas veronii, Pseudomonas moraviensis, Bacillus wiedmannii, Stenotrophomonas tumulicola* and *Bacillus licheniformis* in ratio 1-2:1-2:1-2:1-2:1-2:1-2. The enzymes include acid phosphatase, alkaline phosphatase, alpha-galactosidase, alpha-chymotrypsin, beta-glucosidase, tryptophan deaminase and other. The concentration of the Cellulolytic bacteria and enzymes may be 1-2 x 10⁷ cfu/ml.

According to one example the biostimulant is derived from *Brassica oleracea* species plant extracts. According to another example the biostimulant is concentrated humus extract. According to yet another example, both biostimulants: the one derived from Brassica oleracea species plant extracts and humus extract are used. However, use of other biostimulants may be possible.

The inventors propose a process for anaerobic digestion of food waste comprising hydrolysis, acid forming and methanogenesis, wherein a catalyst is added to the food waste to stimulate microbial activity in anaerobic process, wherein the catalyst comprises a biological agent comprising cellulolytic bacteria and enzymes, wherein the biological agent comprising cellulolytic bacteria consists of cultures *Pseudomonas koreensis, Pseudomonas veronii, Pseudomonas moraviensis, Bacillus wiedmannii, Stenotrophomonas tumulicola* and *Bacillus licheniformis* in ratio 1-2:1-2:1-2:1-2:1-2:1-2.

Further the inventors propose a kit for enhancing anaerobic digestion of food waste and producing biogas. According to one embodiment the kit comprises: (a) a biological agent comprising Cellulolytic bacteria and enzymes, the biological agent consisting of cultures *Pseudomonas koreensis, Pseudomonas veronii, Pseudomonas moraviensis, Bacillus wiedmannii, Stenotrophomonas tumulicola* and *Bacillus licheniformis* in ratio 1-2:1-2:1-2:1-2:1-2:1-2. According to another embodiment the kit further comprises: (b) first biostimulant derived from *Brassica oleracea* species plant extract. According to yet another embodiment the kit further comprises: (c) second biostimulant being a concentrated humus extract. According to one embodiment the components of the kit can be added to a food waste in the following ratio (a):(b):(c) as 1-3:1-3:0.5-2, where the component "(a)" concentration is 1-2 x 10⁷ cfu/ml.

### Brief Description Of Drawings

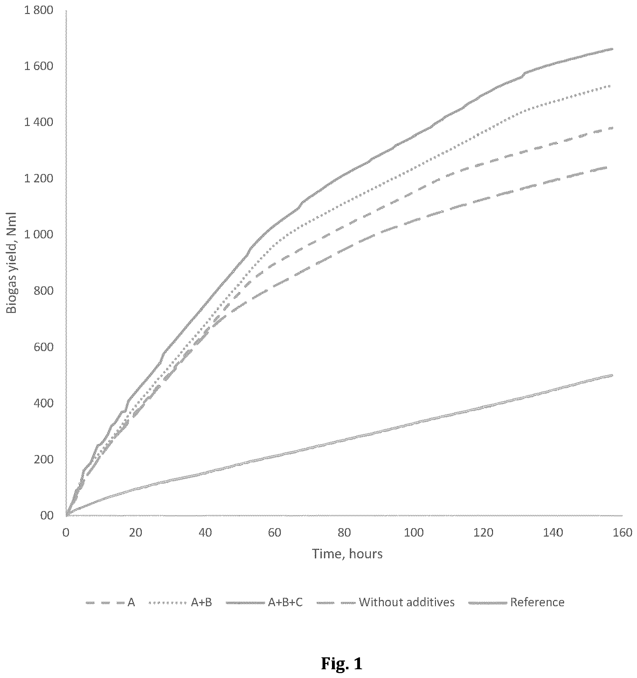

Fig. 1 shows biogas yield with different catalyst combinations according to the present invention, added to the batch type biogas reactors, where 100% organic food waste collected from households and catering in the territory of Riga city was added daily; (a) is a biological agent comprising Cellulolytic bacteria and enzymes; (a+b) - the biological agent comprising Cellulolytic bacteria and enzymes and biostimulant derived from Brassica oleracea species plant extract; (a+b+c) - the biological agent comprising Cellulolytic bacteria and enzymes, biostimulant derived from Brassica oleracea species plant extract as well as a concentrated humus extract. 10 volume % of food waste was added to reactors with inoculum without pH stabilization.

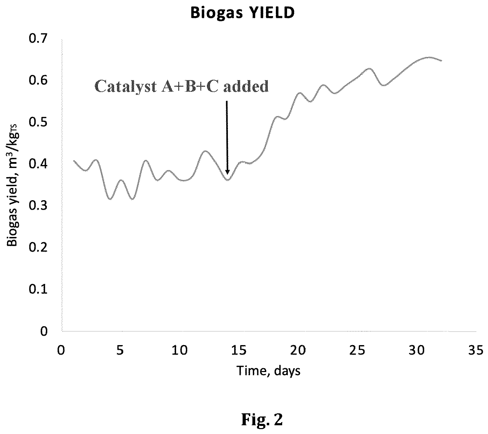

Fig. 2 shows biogas yield in continuous type biogas reactors using catalyst combination (a+b+c), i.e. Cellulolytic bacteria and enzymes, Brassica oleracea species plant extract, as well as a concentrated humus extract.

### Detailed Description Of Invention

The proposed catalyst comprising isolated Cellulolytic bacteria, respective bacterial enzymes and, optionally, one or more biostimulants. Said Cellulolytic bacteria are isolated from organic-polluted soil. Bacteria have been enriched with serial methods using special medium inoculated with the consortium from organic-polluted soil. The catalyst provides not only faster digestion of organic waste, but it activates enzymatic processes to promote rapid cell growth and overcoming limiting conditions that affect microbial activity, therefore, provides more efficient degradation of lignocellulosic substances, which results in higher biogas yield.

The biostimulants according to the invention are specific and targeted non-bacterial concentrates formulated to increase both: the performance of the biological agent comprising Cellulolytic bacteria and resident microorganisms. Especially biostimulants enhance performance of the acid forming and methane producing microorganisms, while microbiological consortium and enzymes are working to enhance activity of hydrolysis.

The extract derived from *Brassica oleracea* species can be obtained by heating method (e.g. at 100° C for 30 minutes, steamed for 20 minutes, cooled and stored at +4° C) or other known method.

The humic substances were isolated from industrially mined raised bog peat using extraction methods. Elemental composition of studied humic substances was detected as follows: C 52%; H 5%, N 1.6%, S 0.5% and ash 2 %. However, tests of other origin humic substances shown comparable results. Applying heating to concentrated humus extract at about 100°C for about 15 minutes is preferable.

The experiments showed surprising synergism of the biological agent comprising said Cellulolytic bacteria. The results showed higher biogas yield using said Cellulolytic bacteria and enzymes together with *Brassica oleracea* species plant extract as a biostimulant. However more effective methanogenesis was observed using the biological agent and both biostimulants together.

Among others, five samples (S1-S5) of the biological agent comprising Cellulolytic bacteria according to the present invention were studied, where different cultures of Cellulolytic bacteria were selected at ratios, shown in Table 1.

**Table 1**

| Samples of biological agent comprising Cellulolytic bacteria studied | | | | | |
|---|---|---|---|---|---|
| Culture | S1 | S2 | S3 | S4 | S5 |
| *Pseudomonas koreensis* | 1 | 2 | 1 | 1 | 0 |
| *Pseudomonas veroni* | 1 | 2 | 1 | 1 | 1 |
| *Pseudomonas moraviensis* | 1 | 2 | 1 | 1 | 0 |
| *Bacillus wiedmannii* | 1 | 1 | 2 | 0 | 2 |
| *Stenotrophomonas tumulicola* | 1 | 1 | 2 | 0 | 0 |
| *Bacillus licheniformis* | 1 | 1 | 2 | 0 | 1 |

Initial study of biological activity of the samples S1, S2, S3, S4 and S5 at 100 mL serum bottles with 5% food waste additive showed that equal microbiological relationship between all species (S1) showed the best performance for anaerobic degradation of food waste in batch reactors, when it is possible to reach biogas yield 0,250-0,305 m³/kg_{total solids}. The lack of some species (as S4 and S5) showed remarkable decrease in process performance, where biogas yield was decreased by 50%. S2 showed similar performance like S1.

Further, samples S1-S3 were tested with different concentration of the biological agent, different biostimulant concentrations and different species of *Brassica oleracea.* The resulting samples K1-K8 are shown in tables 2-5 (the unit KVV/mL in these tables is equivalent to cfu/ml).

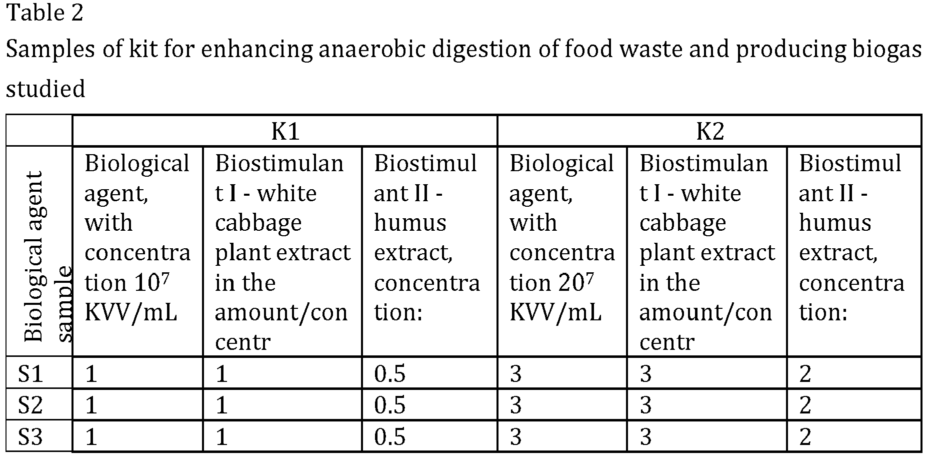

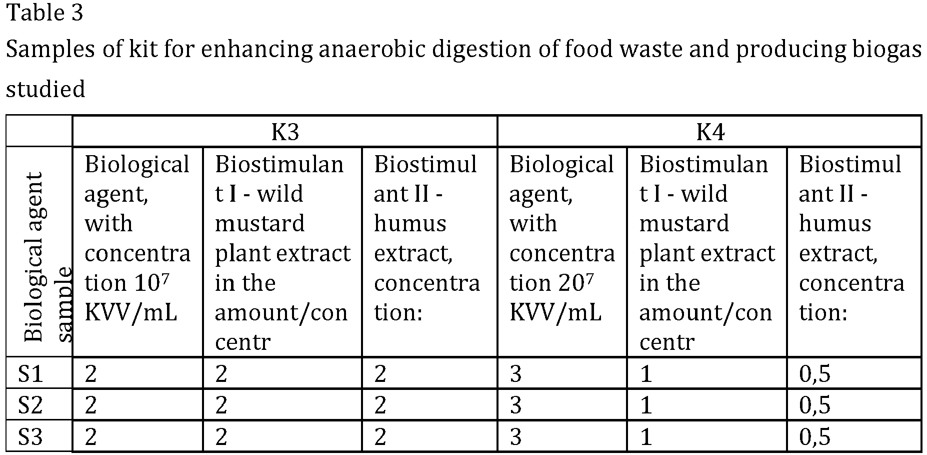

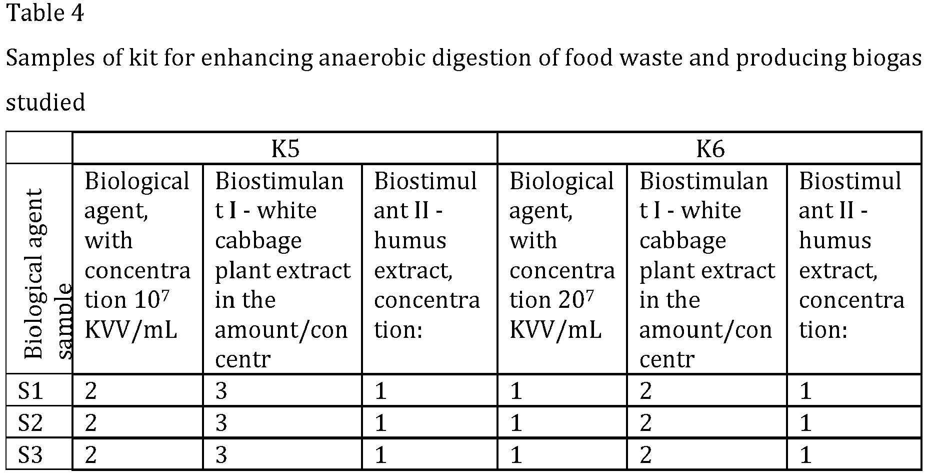

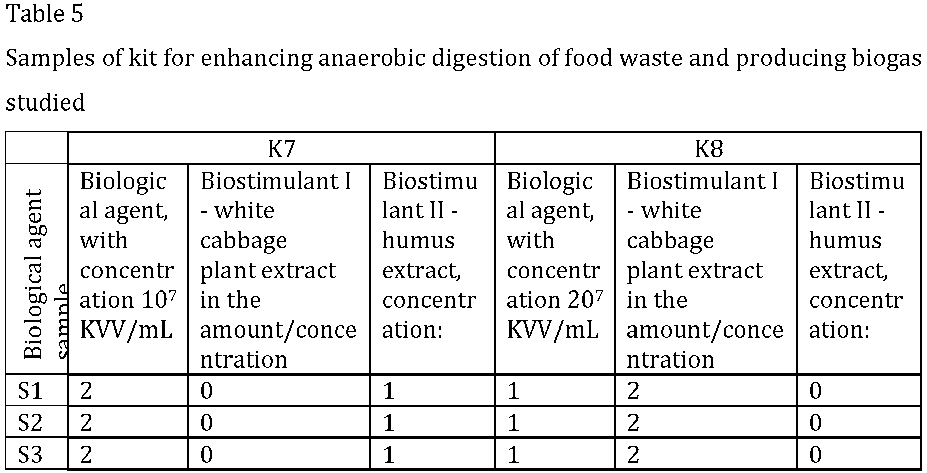

Analysis of K1-K8 showed the best performance of composition K1, where biological agent, biostimulant I (i.e. white cabbage plant extract) and biostimulant II (i.e. humus extract) are added at the following ratio 1:1:0,5, when it is possible to reach biogas yield 0,35-0,37 m³/kg_{total solids} or increase till 40% if 10 volume % of food waste is added to biogas batch-type reactor. However, samples K2-K8 also shown sufficient performance and average increase in biogas production is between 12 - 25% (see Fig.1).

The biological agent comprising Cellulolytic bacteria and enzymes, as well as one or more biostimulants according to the present invention should preferably be added to a waste processed 1-2 times per week. The preferable volume is 4-10 ml/L digester volume, related from total solids content in the biogas reactor. The food waste should preferably consist from 20-40% protein, 0-10% lipids and 40-60% carbohydrates. The total solids should preferably be at range 11-20%. Biogas yield in continuous type biogas reactors using catalyst combination (A+B+C), i.e. Cellulolytic bacteria and enzymes, Brassica oleracea species plant extract, as well as a concentrated humus extract is shown in Fig. 2.

It has been proved that biogas production can be increased significantly by the addition of the catalyst according to the present invention. The catalyst has improved the biogas yield by 20-25% in continuous mode and has decreased FOS/TAC ration (FOS - volatile organic acids, TAC - total inorganic carbon) by 15-30%, which allows increasing organic loading rate for 10-30% during digestion of organic food wastes with total solids content 11-20%. Also, hydraulic retention time was decreased from 56 to 46 days i.e. for about 18%. Approximated increase of biogas produced per annum for industrial biogas station treating food waste is 53%.

The components of the kit for enhancing anaerobic digestion of food waste and producing biogas, comprising: (a) a biological agent comprising Cellulolytic bacteria and enzymes; (b) first biostimulant derived from *Brassica oleracea* species plant extract, and/or (c) second biostimulant being a concentrated humus extract, may be in liquid or in powder form. According to one embodiment the components of the kit are stored in separate containers. However combined storage of the components is possible, subject to storage life limitations.

## Claims

1. A process for anaerobic digestion of food waste comprising hydrolysis, acid forming and methanogenesis, wherein a catalyst is added to the food waste to stimulate microbial activity in anaerobic process, wherein the catalyst comprises a biological agent comprising Cellulolytic bacteria and enzymes; wherein the biological agent comprising Cellulolytic bacteria consists of cultures *Pseudomonas koreensis, Pseudomonas veronii, Pseudomonas moraviensis, Bacillus wiedmannii, Stenotrophomonas tumulicola and Bacillus licheniformis* in ratio 1-2:1-2:1-2:1-2:1-2:1-2.

2. The process according to claim 1, wherein the catalyst further comprises *Brassica oleracea* species plant extract.

3. The process according to claim 2, wherein the catalyst further comprises concentrated humus extract.

4. The process according to any preceding claims, wherein the catalyst comprises: (a) the biological agent comprising Cellulolytic bacteria and enzymes as per claim 1; (b) *Brassica oleracea* species plant extract; (c) a concentrated humus extract; wherein said components of the catalyst are mixed in the following ratios (by volume): a:b:c as 1-3:1-3:0.5-2, where the component "(a)" concentration is 1-2 x 10⁷ cfu/ml.

5. The process according to any preceding claims, wherein the anaerobic digestion of food waste is conducted in a continuous stirrer reactor, where the catalyst is applied to the continuous stirrer reactor one or two times per week in the amount of 4-10 ml/L digester volume.

6. A kit for enhancing anaerobic digestion of food waste and producing biogas, comprising:
(a) a biological agent comprising Cellulolytic bacteria and enzymes,consisting of cultures *Pseudomonas koreensis, Pseudomonas veronii, Pseudomonas moraviensis, Bacillus wiedmannii, Stenotrophomonas tumulicola and Bacillus licheniformis* in ratio 1-2:1-2:1-2:1-2:1-2:1-2.

7. The kit according to claim 6, further comprising: (b) *Brassica oleracea* species plant extract.

8. The kit according to claim 7, further comprising: (c) a concentrated humus extract.

## Patentansprüche

1. Verfahren zur anaeroben Zersetzung von Lebensmittelabfällen durch Hydrolyse, Säurebildung und Methanogenese, wobei den Lebensmittelabfällen ein Katalysator zugegeben wird, um die mikrobielle Aktivität in einem anaeroben Verfahren zu stimulieren. Der Katalysator besteht aus einem biologischen Wirkstoff, der aus cellulolytischen Bakterien und Enzymen zusammengesetzt ist. Dabei besteht der cellulolytische Bakterien enthaltende biologische Wirkstoff aus Kulturen von *Pseudomonaskoreensis, Pseudomonas veronii, Pseudomonas moraviensis, Bacillus wiedmannii, Stenotrophomonas tumulicola* und *Bacillus licheniformis* im Verhältnis 1-2:1-2:1-2:1-2:1-2:1-2.

2. Verfahren nach Patentanspruch 1, wobei der Katalysator außerdem noch den Pflanzenextrakt der Art *Brassica oleracea* enthält.

3. Verfahren nach Anspruch 2, wobei der Katalysator außerdem noch konzentrierten Humusextrakt enthält.

4. Verfahren nach einem der vorhergehenden Ansprüche, wobei der Katalysator: (a) ein biologischer Wirkstoff aus cellulolytischen Bakterien und Enzymen nach Anspruch 1; (b) ein Pflanzenextrakt der Art *Brassica oleracea*; (c) ein konzentrierter Humusextrakt ist. Dabei werden die Komponenten des Katalysators in den folgenden Verhältnissen (nach Volumen) gemischt: a:b:c als 1-3:1-3:0,5-2, wobei die Konzentration der Komponente "(a)" 1-2 x 107cfu/ml beträgt.

5. Verfahren nach einem der vorhergehenden Ansprüche, in dem die anaerobe Vergärung von Lebensmittelabfällen in einem Rührkesselreaktor durchgeführt wird, wobei der Katalysator ein oder zweimal pro Woche in einer Menge von 4-10 ml/l Fermentervolumen in den Rührkesselreaktor gemischt wird.

6. Ein Bausatz zur Verbesserung der anaeroben Vergärung von Lebensmittelabfällen und zur Erzeugung von Biogas, bestehend aus:
(a) einem biologischen Wirkstoff, der sich aus cellulolytischen Bakterien und Enzymen zusammensetzt - den Kulturen von *Pseudomonas koreensis, Pseudomonas veronii, Pseudomonas moraviensis, Bacillus wiedmannii, Stenotrophomonas tumulicola* und *Bacillus licheniformis* in einem Verhältnis von 1-2:1-2:1-2:1-2:1-2:1-2.

7. Der Bausatz nach Anspruch 6, ferner bestehend aus: (b) einem Pflanzenextrakt der Art *Brassica oleracea.*

8. Der Bausatz nach Anspruch 7, ferner bestehend aus: (c) einem konzertierten Humusextrakt.

## Revendications

1. Procédé pour la digestion anaérobie de déchets alimentaires comprenant une hydrolyse, une formation d'acide et une méthanogenèse, dans lequel un catalyseur est ajouté aux déchets alimentaires pour stimuler une activité microbienne dans un procédé anaérobie, dans lequel le catalyseur comprend un agent biologique comprenant des bactéries cellulolytiques et des enzymes ; dans lequel l'agent biologique comprenant des bactéries cellulolytiques est constitué des cultures *Pseudomonas koreensis, Pseudomonas veronii, Pseudomonas moraviensis, Bacillus wiedmannii, Stenotrophomonas tumulicola* et *Bacillus licheniformis* dans un rapport 1-2:1-2:1-2:1-2:1-2:1-2.

2. Procédé selon la revendication 1, dans lequel le catalyseur comprend en outre un extrait végétal de l'espèce *Brassica oleracea.*

3. Procédé selon la revendication 2, dans lequel le catalyseur comprend en outre de l'extrait d'humus concentré.

4. Procédé selon l'une quelconque des revendications précédentes, dans lequel le catalyseur comprend : (a) l'agent biologique comprenant des bactéries cellulolytiques et des enzymes selon la revendication 1 ; (b) un extrait végétal de l'espèce *Brassica oleracea* ; (c) un extrait d'humus concentré ; dans lequel lesdits composants du catalyseur sont mélangés dans les rapports suivants (en volume) : a:b:c selon 1-3:1-3:0,5-2, où la concentration en composant « (a) » est de 1-2 x 10⁷ ufc/mL.

5. Procédé selon l'une quelconque des revendications précédentes, dans lequel la digestion anaérobie de déchets alimentaires est effectuée dans un réacteur à agitation continue, où le catalyseur est appliqué au réacteur à agitation continue une ou deux fois par semaine dans la quantité de 4 à 10 mL/L de volume de digesteur.

6. Trousse pour améliorer une digestion anaérobie de déchets alimentaires et produire du biogaz, comprenant :
(a) un agent biologique comprenant des bactéries cellulolytiques et des enzymes, constitué des cultures *Pseudomonas koreensis, Pseudomonas veronii, Pseudomonas moraviensis, Bacillus wiedmannii, Stenotrophomonas tumulicola* et *Bacillus licheniformis* dans un rapport 1-2:1-2:1-2:1-2:1-2:1-2.

7. Trousse selon la revendication 6, comprenant en outre : (b) un extrait végétal de l'espèce *Brassica oleracea.*

8. Trousse selon la revendication 7, comprenant en outre : (c) un extrait d'humus concentré.
